(19) **Europäisches Patentamt European Patent Office Office européen des brevets**

(11) **EP 3 638 102 B1**

(12) **EUROPEAN PATENT SPECIFICATION**

(45) Date of publication and mention of the grant of the patent:
**23.08.2023 Bulletin 2023/34**

(21) Application number: **18732443.9**

(22) Date of filing: **08.06.2018**

(51) International Patent Classification (IPC):
***A61B 5/00*** *(2006.01)*    ***A61B 5/053*** *(2021.01)*
***A61N 1/05*** *(2006.01)*

(52) Cooperative Patent Classification (CPC):
**A61B 5/4029; A61B 5/0538; A61B 5/6877;**
A61N 1/0556

(86) International application number:
**PCT/GB2018/051571**

(87) International publication number:
**WO 2018/229463 (20.12.2018 Gazette 2018/51)**

(54) **DETECTING ACTIVITY IN PERIPHERAL NERVES**

ERKENNUNG EINER AKTIVITÄT IN PERIPHEREN NERVEN

DÉTECTION D'UNE ACTIVITÉ DANS LES NERFS PÉRIPHÉRIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.06.2017 GB 201709666**

(43) Date of publication of application:
**22.04.2020 Bulletin 2020/17**

(73) Proprietor: **Cyqiq Ltd**
**London N2 0QX (GB)**

(72) Inventors:
• **DOWRICK, Thomas**
**London N1 4NW (GB)**
• **ARISTOVICH, Kirill**
**London N7 8DL (GB)**
• **HOLDER, David**
**London N2 0QX (GB)**

(74) Representative: **Boult Wade Tennant LLP**
**Salisbury Square House**
**8 Salisbury Square**
**London EC4Y 8AP (GB)**

(56) References cited:
**EP-A1- 2 543 311      WO-A1-2016/170327**
**WO-A1-2017/030900**

• **HOLDER D S ED - ZARKOGIANNI KONSTANTIA ET AL: "IMPEDANCE CHANGES DURING THE COMPOUND NERVE ACTION POTENTIAL: IMPLICATIONS FOR IMPEDANCE IMAGING OF NEURONAL DEPOLARISATION IN THE BRAIN", MEDICAL AND BIOLOGICAL ENGINEERING AND COMPUTING, SPRINGER, HEILDELBERG, DE, vol. 30, no. 2, 1 March 1992 (1992-03-01), pages 140-146, XP000271685, ISSN: 0140-0118, DOI: 10.1007/BF02446122**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 638 102 B1

## Description

The present invention relates to apparatus for detecting activity in a peripheral nerve of a human or animal subject.

## Introduction

Methods and apparatus for detecting electrical activity and properties in a peripheral nerve are discussed in WO2016/170327, which discusses techniques for carrying out electrical impedance tomography. The duration of a single action potential of a peripheral nerve is of the order of a few milliseconds, and it would therefore be desirable to be able to carry out electrical impedance tomography fast enough to image a single such action potential event.

EP 2 543 311 describes a system and method for excitation generation in soft-field tomography. D.S.Holder, Medical & Biological Engineering & Computing, 30, March 1992, pages 140 - 146, discusses measurements of impedance changes during the compound action potential in isolated crab nerves. WO2017/030900 proposes a quantitative tool using impedance spectroscopy to monitor fracture healing.

## Summary of the invention

The invention provides apparatus for determining electrical properties within a peripheral nerve of a human or animal subject, as set out in the claims.

Embodiments of the invention can be used for imaging a cross section of electrical activity within nervous tissue such as a peripheral nerve, using phase multiplexed electrical impedance tomography in which multiple probe electrical signals of different phases are applied to different combinations of electrodes at the same time. Frequency multiplexing may also be used to apply multiple probe electrical signals of both different phases and different frequencies at the same time. Embodiments provide apparatus arranged to carry out such phase multiplexed and/or frequency multiplexed electrical impedance tomography.

Embodiments of the invention may for example use from two to five, and typically three frequency channels, and two phase channels, the frequency channels typically being spaced by around 3 kHz from each other within an operating range from about 2 kHz or 5 kHz up to about 12 kHz (although other operating ranges for example within an operating range from 1 to 20 kHz may be used), and the phase channels typically being in quadrature with phase spacing of about $\pi/2$. In this way, a single frame or image of a cross section of the nerve formed using impedance tomography can represent a time interval of a few milliseconds or less.

More particularly, embodiments of the invention may be used to determine electrical properties in a peripheral nerve of a human or animal subject, by spacing a plurality of electrodes around a perimeter of the peripheral nerve, simultaneously applying a phase separated pair of first and second probe electrical signals to a set of respective first and second combinations of electrodes, the phase separated pair of first and second probe electrical signals having different signal phases from each other, collecting one or more combined responses to the phase separated pair at one or more of the plurality of electrodes, detecting a pair of phase separated electrical responses corresponding to the different signal phases from the one or more combined responses, and using the pair of phase separated electrical responses to determine electrical properties of the peripheral nerve within the perimeter.

The or each phase separated pair of probe electrical signals may comprise first and second probe electrical signals which are substantially in phase quadrature with respect to each other, or which at least have orthogonal phase components which can be separated from each other.

In this way, using two phase channels for the probe electrical signals and detected electrical responses, the rate of interrogation of the nerve can be doubled, or the time taken to acquire the data for a single frame can be halved. The or each phase separated pair of probe electrical signals may comprises first and second probe electrical signals which are substantially in phase quadrature with respect to each other, for example to optimise the detection of the two phase channels.

One or more other multiplexing schemes can be used at the same time to further reduce the required acquisition time for a single frame, for example by applying each of a plurality of said phase separated pairs of probe electrical signals to a different set of combinations of electrodes, such that collecting one or more combined responses comprises collecting one or more combined responses to the phase separated pairs at one or more of the plurality of electrodes; detecting a pair of phase separated electrical responses corresponding to each phase separated pair of probe electrical signals from the one or more combined responses; and using the plurality of pairs of phase separated electrical responses to determine electrical properties of the peripheral nerve within the perimeter.

In particular, frequency division multiplexing may be used wherein a frequency multiplexed plurality of said phase separated pairs of probe electrical signals are applied simultaneously to each other, each such phase separated pair comprising probe electrical signals of a different frequency band, and the step of detecting comprises detecting a pair of phase separated electrical responses corresponding to each frequency band from the one or more combined responses.

In order to obtain adequate quality measurements of impedance of a peripheral nerve, the different frequency bands may comprise from three to five different frequency bands, for example with the centres of all of the frequency bands lying in the range from 1 to 20 kHz, from 2 to 12 kHz, or from 5 kHz to 12 kHz.

Determining electrical properties of the peripheral nerve within the perimeter may comprise carrying out an electrical impedance tomography image reconstruction using the electrical responses, to determine electrical properties at one or more locations within the perimeter, or more particularly to determine a map or image of electrical properties of a cross section of the peripheral nerve, although other analysis techniques may also or instead be applied to generate useful information about nerve activity or structure from the electrical response data. Typically, the determined electrical properties will be representative of impedance, although other suitable electrical properties may be represented.

Embodiments of the invention may be applied to monitoring or detecting structure and/or activity in a peripheral nerve, for example in particular in an autonomic nerve.

Spacing the plurality of electrodes around a perimeter of the peripheral nerve (or other structure) may comprise providing the plurality of electrodes on a flexible cuff and wrapping the flexible cuff around the perimeter of the nerve so that the electrodes are in contact with the perimeter of the peripheral nerve.

Embodiments of the invention also provide apparatus comprising a signal source arranged to simultaneously apply a phase separated pair of first and second probe electrical signals to a set of respective first and second combinations of electrodes, the phase separated pair of first and second probe electrical signals having different signal phases from each other; and a detector arranged to receive one or more combined responses to the phase separated pair at one or more of the plurality of electrodes and to detect a pair of phase separated electrical responses corresponding to the different signal phases from the one or more combined responses.

The apparatus may be provided with a switch, arranged to complete required connections between the signal source and the electrodes of the nerve cuff, and optionally between the detector and the electrodes of the nerve cuff nerve cuff, the switch being programmed or controlled to cycle through various connection configurations required for the electrical impedance tomography procedure.

Such apparatus may also comprise a nerve cuff arranged to space a plurality of electrodes around a perimeter of the peripheral nerve, although such a nerve cuff may often be provided as a separate element for connection to the signal source and detector. Such a nerve cuff may comprise a flexible substrate for wrapping around at least part of a perimeter of a peripheral nerve, and the plurality of electrodes may then be spaced on or in the flexible substrate for contacting the peripheral nerve around the perimeter. Electrical connections may then be provided to each of the electrodes for applying the probe electrical signals from the signal source, and for collecting the combined responses.

Apparatus according to embodiments of the invention may also provide a computing element arranged to use the phase separated electrical responses, and the frequency separated responses made if available, to determine electrical properties within the peripheral nerve using electrical impedance tomography image construction.

Apparatus for implementing the invention may be adapted for implantation in the human or animal subject, and such apparatus for implantation may also comprise suitably programmed computer hardware for carrying out analysis such as tomographic image construction. Alternatively, some or all of the apparatus, especially the data processing and analysis functionality may be provided externally to the nerve cuff and any other parts of the equipment implanted in a subject.

## Brief description of the drawings

Embodiments of the invention will now be described, by way of example only, with reference to the drawings, of which:

Figure 1 illustrates, in use, a device for monitoring a peripheral nerve of a human or animal subject;
Figure 2 illustrates schematically functional elements which may be provided in the controller of figure 1, and/or elsewhere including externally to the subject, in order to determine electrical properties of a nerve according to embodiments of the invention;
Figure 3 provides a graph of experimental signal to noise ratio of impedance measurements of a peripheral nerve derived using probe electrical signals of different frequencies;
Figure 4 illustrates frequency channels suitable for use in electrical impedance tomography of a peripheral nerve;
Figure 5 is a schematic illustration of simultaneous phase division and frequency division multiplexing in electrical impedance tomography of a peripheral nerve;
Figure 6 illustrates a practical laboratory implementation of apparatus arranged to implement embodiments of the invention; and
Figure 7 shows how a signal source for use in embodiments of the invention to form a probe electrical signal may be implemented.

## Detailed description of embodiments

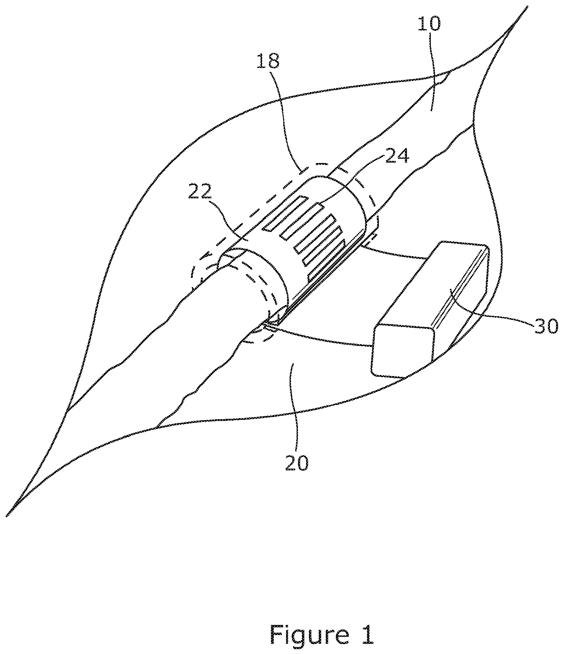

Referring to figure 1 there is shown in perspective view a peripheral nerve 10 of a human or animal subject, to which an example nerve monitoring device 20 has been coupled. The nerve monitoring device is arranged to use electrical impedance tomography to detect electrical activity within the nerve, through changes in the electrical properties within the nerve, in particular to detect such activity at one or more locations within a cross section of the nerve at the location of the device. An image or map of such electrical properties over a cross section through the nerve may be derived. The electrical properties may typically correspond to or be impedance.

Embodiments of the invention may operate at least in part by measuring the change in resistance produced by the opening of ion channels in the membranes of peripheral nerves as they fire. Probe current applied to the nerve using an externally applied probe electrical signal travels in the extracellular space of the nerve when a nerve fibre is at rest, because in this state nerve fibre membranes have a very high resistance. As ion channels open during the action potential of a nerve fibre, the externally applied probe current travels into the intracellular compartment of the fibre which contains additional conducting ions. This lowers the resistance of the bulk tissue by about 1% at DC, and typically less with increasing frequency of the applied current. Other mechanisms may also be effective in changing the apparent impedance or other electrical properties within the peripheral nerve which are evident from or can be derived from the electrical responses at the surface of the nerve to an applied electrical signal.

In figure 1 the nerve 10 is shown as surgically exposed, but the device 20 may typically be surgically implanted in a permanent or temporary manner. The device 20 comprises a nerve cuff 22 which wraps around an outside perimeter of the nerve 10 and which is provided with a plurality of electrodes 24 for contacting the nerve, preferably without needing to penetrate into the nerve tissue, although some degree of penetration may occur, for example due to pressure exerted by the cuff, or be desirable, for example to reduce contact impedance. In the figure, the electrodes 24 are drawn, but in practice would usually be hidden beneath the material of the cuff 22. In figure 1 the device also comprises an associated control unit 30 coupled to and located proximal to the cuff. Control functionality to support use of the cuff 22 and the electrodes 24 may be provided solely within the control unit 30, or partly or solely in one or more other units located within and/or external to the subject, for example in external electronics and computer equipment, or in a mixture of these options.

The cuff 22 may be held in place around the nerve in a variety of ways. In figure 1 a clamp in the form of an elastomeric tube 18 having a slot allowing a portion of the cuff to extend away from the nerve towards the control unit 30 has been used. The clamp then holds an interior surface of the cuff, on which the electrodes are exposed, against the periphery of the nerve. More generally, the clamp may be designed in such a way that it holds the array, which is wrapped around the nerve on the side proximal to the control unit, holding and pressing both tails of the array against each other. The clamp is designed in a way that the force, applied to the nerve from the cuff, is limited so it is impossible to damage the nerve during normal operation.

Although a particular nerve cuff 22 and associated components has been described briefly above, a variety of other nerve cuff arrangements such as those already known in the prior art may be used to implement the methods and apparatus described elsewhere in this document, for example nerve cuff structures which comprise single or multiple flexible or rigid elements to be disposed around a nerve in various geometries and combinations, and electrodes which contact without penetrating or which penetrate into the surface of the nerve. Electrodes for contacting or penetrating the surface of a nerve may be made of a variety of materials including various metals, semiconductors, carbon materials and so forth. Typically the number of electrodes used may be from about sixteen to 64, and the nerve cuff may be sized and configured to wrap around or otherwise encircle any chosen peripheral nerve or nerve size, for example from about 0.5 mm to about 3.0 mm in diameter.

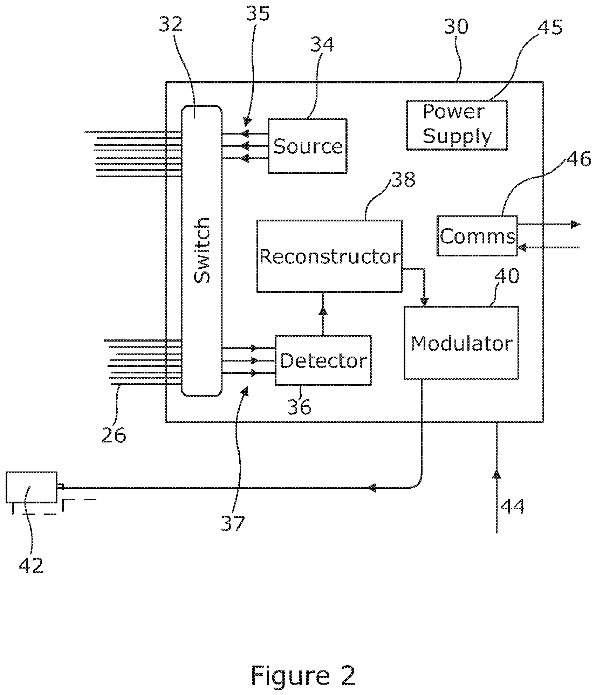

Aspects of the example control unit 30 of figure 1 are shown schematically in figure 2. Electrical connections 26 between the control unit 30 and the electrodes 24 of the nerve cuff 22 are coupled to a switch 32 which allows particular electrodes 24, combinations of electrodes, and sets of such combinations of electrodes, to be selected and coupled to other parts of the control unit 30. The signal source 34 can then be used to deliver a particular probe electrical signal 35 to any particular combination of electrodes, and indeed to deliver multiple different probe electrical signals 35 simultaneously to multiple different combinations of electrodes. As described in more detail below, such simultaneous probe electrical signals 35 may differ, and be distinguishable from each other, by being of different frequencies and/or phases, which may be referred to herein as different frequency and phase channels.

A detector 36 is then used to measure electrical responses collected at one or more of the electrodes at the same time as the probe electrical signals are being delivered, through connection to the detector by the switch 32. In particular, such electrical responses may be collected from each of a plurality of combinations of the electrodes at the same time. Since the electrical response collected from a particular combination such as a particular pair of electrodes will typically include contributions resulting from the probe electrical signals of different frequencies and/or phases being applied simultaneously as mentioned above, each such electrical response collected from a particular combination of electrodes may be referred to as a combined electrical response 37. The detector 36 then applies frequency and/or phase detection or filtering in order to separate out the electrical responses on the different frequency and/or phase channels.

Typically, the probe electrical signals generated by the signal source 34 are alternating current signals, and the collected electrical responses are alternating voltages at each of a plurality (some or all) of the other electrodes 24 which are in contact with the nerve but not used for applying that particular probe signal. For example, electrical responses may be collected from all possible combinations of such non-probe electrodes, or a particular subset of those combinations. The collected electrical responses taken in combination with the corresponding probe signal, for each of multiple probe signals applied to different combinations of electrodes, then allow an impedance or conductance map within the ring of electrodes to be derived. The probe electrical signals may be AC or DC, although in practice AC is nearly always used. The probe electrical signals are usually current signals, so that the measured resulting electrical responses are voltages, but the probe electrical signals may be voltage signals and the resulting electrical responses are then current signals. In either case, the resulting electrical responses represent a measure of impedance within the peripheral nerve.

The probe electrical signals may typically be narrowband sinusoidal signals in which substantially all of each such signal, or a majority of each such signal, in terms of amplitude, power or another suitable measure, is found at a frequency within the specified range, or at a range of frequencies within the specified range.

The probe electrical signal should preferably give rise to currents within the peripheral nerve which do not cause action potentials to be produced or significantly alter the shape of the compound action potential or its component elements. In other words, the probe signal should not significantly alter behaviour of the monitored peripheral nerve. The limit on such currents within the nerve so as not to affect nerve behaviour may depend on frequency of the probe signal.

In particular, the monitoring device 20 may be used to measure transfer impedances using a four electrode method. In such a method a particular probe electrical signal is applied to two of the electrodes, and the resulting electrical responses are measured between each of a plurality of different pairs of other electrodes. These pairs may be measured one by one in series, but in order to improve rate of detection are preferably measured together at the same time in parallel. Using a four electrode method and transfer impedances avoids having to take into account the contact impedances of the electrodes delivering the probe electrical signals. Of course, more than two electrodes can be used to apply the probe signal, for example combinations of larger numbers of electrodes in a desired spatial pattern.

The measured electrical responses as signals of AC (or sometimes DC) voltage or current then correspond to transfer impedances, and comprise the carrier frequency of the probe signal modulated over time by changes in impedance in the peripheral nerve. The voltages or currents of the electrical responses are therefore converted to impedance signals by demodulation with respect to the carrier frequency of the applied AC signal to give a complex impedance which varies over time. Different aspects of this complex impedance may be used to derive the required electrical properties of the nerve. Usually, the real components of the measured transfer impedances are used, because this is least contaminated by stray capacitance. However, any property of the complex impedance such as phase angle, modulus, or quadrature component may be used. Electrical properties of the peripheral nerve determined from the measured electrical responses may be generated in an absolute form, or more usually at a difference over time or applied frequency.

The control unit 30 may also comprise a reconstructor element 38 which is arranged to carry out an electrical impedance tomography reconstruction of the resulting electrical responses, to thereby derive corresponding electrical properties, typically corresponding to impedance, at a plurality of locations within the cross section of the nerve around which the array of electrodes is disposed. These derived electrical properties of the nerve then correspond to nerve activity at the plurality of locations. The reconstructor 38 may be arranged to provide a map or image of the electrical properties across the cross section of the nerve, or may be arranged to provide the electrical properties at one or more selected points or in one or more selected regions of the nerve cross section. Also, instead of being provided as part of the control unit, tomographic reconstruction may be provided by an external entity separate to the control unit 30. The resulting data may demonstrate nerve activity at various levels of resolution, for example in particular geometric parts, particular fascicles, other particular groups of nerve fibres, and even in particular nerve fibres.

Although the reconstructor 38 may be used to generate an electrical impedance tomography map or image in cross section through the nerve, the measured electrical responses may be used in other ways. For example, a mapping technique may be used in which information from the responses is used more directly, as a map onto the surface of the peripheral nerve. In other examples, machine learning, and other classifier and statistical techniques may be used to identify patterns of activity within the nerve without requiring reconstruction of a tomographic image. These examples may provide useful results from the electrical responses more quickly than is possible using a full tomographic image reconstruction.

The control unit may also comprise a modulator 40 which is arranged to apply a modulation signal to the peripheral nerve 10 to modulate activity within the nerve, the modulation signal being generated and applied dependent upon the detected electrical properties or activity of the nerve at one or more locations or in one or more regions as determined by the reconstructor 38. This modulation signal could for example be applied to the nerve using one or more of the electrodes 24, or using one or more additional electrodes 42, or in some other way.

The control unit 30 may also comprise a power supply 45, for supplying power to the other elements of the control unit described herein, for example using a battery or similar. As already mentioned, one or more functions of the control unit 30 may instead or additionally be provided in an additional unit located proximal to or distal from the device 20, inside or outside the human or animal body in which the device is implemented.

Aspects of the control unit 30 and related functionality described herein may be implemented using one or more microprocessors with associated memory for storing programs and data. For example, the functionality of the reconstructor 38 to carry out the electrical impedance tomography reconstruction may be carried out in software using such a microprocessor.

The control unit 30 may also be provided with a communications interface 46 for inputting and/or outputting data and/or control signals, for example using a wired or wireless link. Some uses of such a control unit include outputting determined electrical properties of the nerve being tested, for example as an electrical impedance tomography map or image.

Implementations of the invention may provide an implantable device having a cuff as described with around 16-64 electrodes, and appropriate functionality in the control unit to provide electrical impedance tomography maps or other data at various rates as required, for example at about 1 kHz. Such a device may be powered using a battery and operate using a microprocessor for control of the switch 32, signal source 34, detector 36, and implementation of the reconstructor function 38.

Although figures 1 and 2 illustrate the various functionality to support use of the cuff 22 and the electrodes 24 being provided within a discrete the control unit 30, any or all of the associated functions may instead or as well be carried out in other units or apparatus, implanted in or external to the human or animal body comprising the nerve to be monitored.

The duration of an action potential at a particular point in a single nerve fibre of a peripheral nerve is of the order of a few milliseconds, and it would therefore be desirable to be able to image a peripheral nerve with a comparable time resolution. To obtain measured electrical responses from a sufficiently large number of combinations of electrodes to which the probe electrical signals are applied and a sufficient number of different collection electrode pairs or combinations so as to construct a single tomographic image, prior art experimental systems have frequently relied on using an externally or artificially induced nerve impulse which is repeated, for example at a frequency of around 1 Hz. Probe electrical signals are then applied to a just one or a limited number of electrode combinations at each repetition, and perhaps also only a subset of the required collection geometries are used at each repetition, so that the required measurements are built up over a much longer time frame.

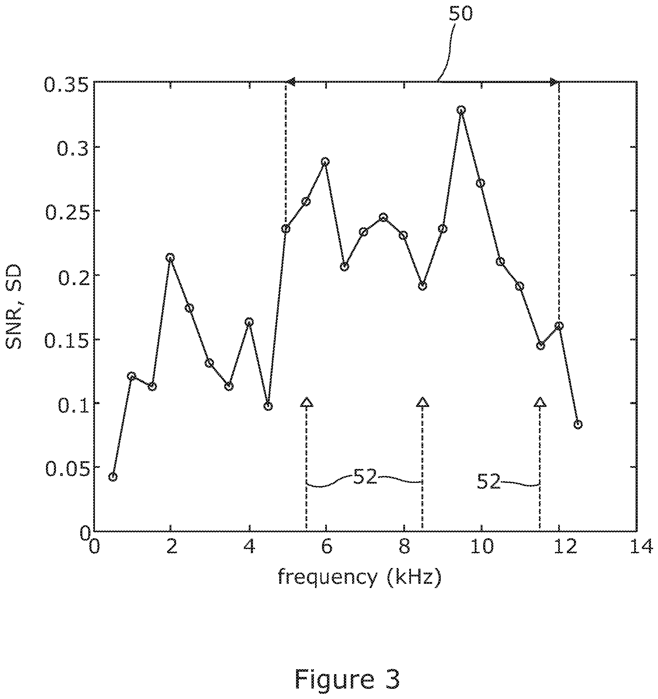

To image nerve activity occurring naturally without artificial stimulation and repetition, it is desirable to apply probe electrical signals to all of the required different combinations of electrodes in just a few milliseconds at most. However, as illustrated in figure 3, the inventors have found that a further constraint when working with a peripheral nerve of a human or animal subject is that the electrical frequency of a probe electrical signal is preferably between about 1 kHz and 20 kHz, and more preferably between about 2 kHz and 12 kHz, or 5 kHz and 12 kHz, in order to yield a reasonably good measure of impedance in the detected electrical response. Figure 3 shows standard deviations of a signal to noise ratio of detected electrical responses in a sheep vagus nerve in response to action potentials as detected using probe electrical signals at a range of probe signal frequencies. A workable frequency range for a probe electrical signal may be said to be from about 1 kHz to 20 kHz, of from 2 kHz to 12 kHz, and a more optimal range from about 5 kHz to about 12 kHz. One such optimal frequency range is labelled 50 in figure 3.

Assuming that about four cycles of a probe electrical signal waveform are required in order to collect a corresponding electrical response, a probe frequency of 5 kHz is consistent with detecting an action potential in a nerve lasting a few milliseconds. However, if a time division multiplexing scheme is then used whereby a probe electrical signal is applied to each of multiple combinations of electrodes in turn, this frequency limitation may only permit application of a probe signal to one or perhaps two different electrode combinations within the duration of the action potential, which is not enough to be able to carry out a tomographic reconstruction of electrical activity within the nerve.

To increase the number of different combinations of electrodes which may be probed within the duration of a single action potential, frequency division multiplexing may be used, whereby a plurality of probe electrical signals of different frequencies are applied to different combinations of electrodes at the same time. Collected combined electrical responses including all of the different frequencies can then be analysed to detect separate electrical responses for each of the frequency channels and therefore for multiple combinations of electrodes at the same time. This may be referred to as a frequency multiplexing scheme.

The inventors have determined that to obtain optimal or adequate information from a detected electrical response in a peripheral nerve based on a single frequency probe signal, a detection bandwidth of at least 2 kHz, and more preferably about 3 kHz may be required. On this basis, only about three or four frequency channels can be used simultaneously within a typical workable frequency range for peripheral nerves discussed above and illustrated in figure 3, where one appropriate selection of frequencies 52 for probe electrical signals is illustrated as 5.5 kHz, 8.5 kHz and 11.5 kHz.

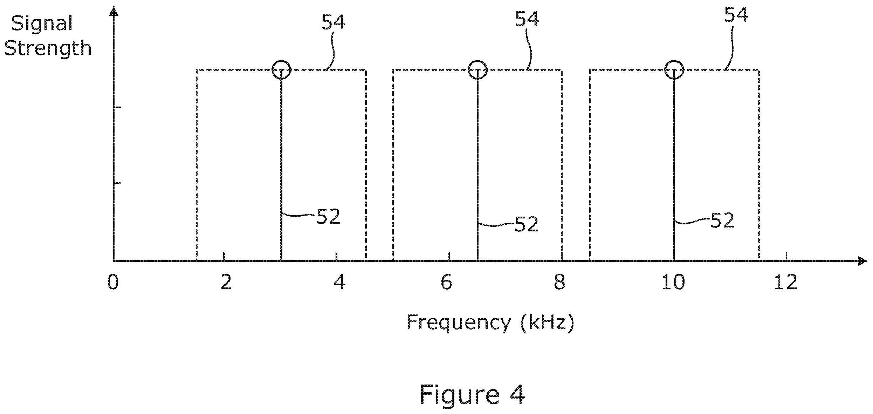

In figure 4 another such selection is illustrated as 3 kHz, 6.5 kHz and 10 kHz. Assuming a bandwidth of a collected electrical response of 3 kHz, this advantageously provides frequency channels 54 for the collected signals of 3 kHz bandwidth with an additional 0.5 kHz guard band between each to further help avoid any cross talk between the channels which could lead to reduced signal to noise ratio.

However, using multiple frequency channels as discussed above still leaves the rate at which different combinations of electrodes can be probed being rather low for the purpose of constructing adequate tomographic images of single action potential events lasting only a few milliseconds. This rate can be increased significantly by using a phase division multiplexing scheme in which a phase separated pair of first and second probe electrical signals is applied simultaneously to respective first and second combinations of electrodes. The first and second probe electrical signals are preferably of the same or similar frequencies, for example at least within about 0.1 kHz, and preferably within about 10 Hz from each other but distinguishable by having different phases which enable the resulting electrical responses to be separated. For optimal phase separation and hence best signal to noise ratio the phase difference between the phase separated probe electrical signals should be $\pm \pi / 2$, that is in quadrature, but some variation from this ideal phase difference is possible, and indeed might be desirable to accommodate slight phase shifts in the signal which occur due to electrical properties of the nerve and the electrodes, for example where phase response of the nerve and/or other parts of the system is known.

Either of the above frequency division multiplexing and phase division multiplexing schemes can be used in isolation, or they can be used in combination, to shorten the time required to apply probe electrical signals to all of the required different combinations of electrodes, and therefore improve the resolution for detection of electrical properties within the peripheral nerve such as a tomographic maps of impedance.

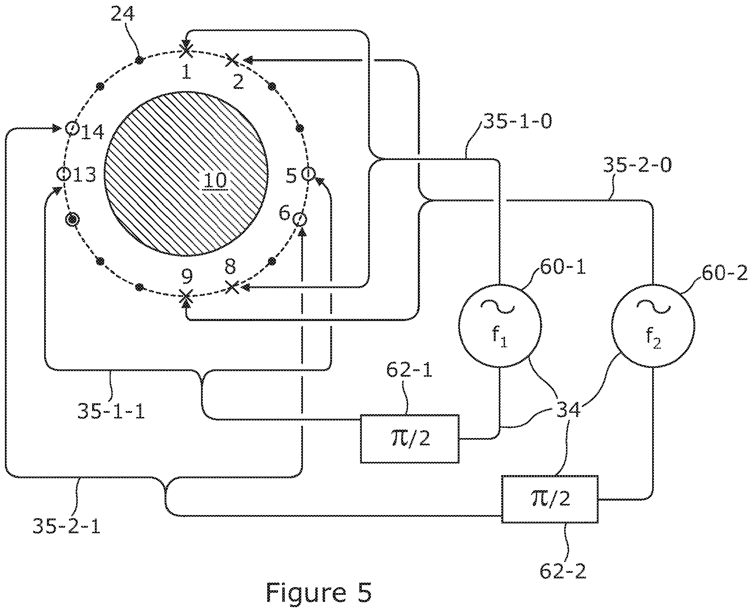

Figure 5 illustrates how either or both of the frequency division and phase division multiplexing schemes may be applied to determine electrical properties in a peripheral nerve 10 of a human or animal subject with a higher time resolution. The nerve 10 is surrounded by a plurality of electrodes 24 disposed in a ring around the nerve 10, for example as already illustrated in figure 1. A signal source 34 comprises a first and second frequency generators 60-1 and 60-2, each generating a probe electrical signal of a different frequency $f_1$, $f_2$, for example at 5.5 kHz and 8.5 kHz as illustrated in figure 3. Each signal source 60-1, 60-2 can be coupled to the electrodes either with or without passing through a phase delay element 62-1, 62-2 of $\pm\pi/2$, typically also included in the signal source 34. In practice, at least one further frequency channel $f_3$ would typically be used, but is not illustrated here for clarity.

Probe signals of the two different frequencies $f_1$, $f_2$ at each of the two different phases can be coupled to any particular required combination of electrodes using a switch 32 such as that shown in figure 2. In figure 5 this is illustrated as particular connections at a particular instant in time, with the four different probe electrical signals 35-1-0, 35-2-0, 35-1 - 1, 35-2-1 coupled across the following pairs of electrodes respectively: (1,8), (2,9), (5,13), (6,14). This particular switch configuration may be held for a time interval, say of a few hundred microseconds, before a second and possibly further different switch configurations are used.

For each switch configuration coupling the different frequency and probe electrical signals to the electrodes, electrical responses are collected for each of multiple pairs or groups of electrodes. These electrical responses combine signals from the different frequency and phase channels, which can then be separated out in the detector 36.

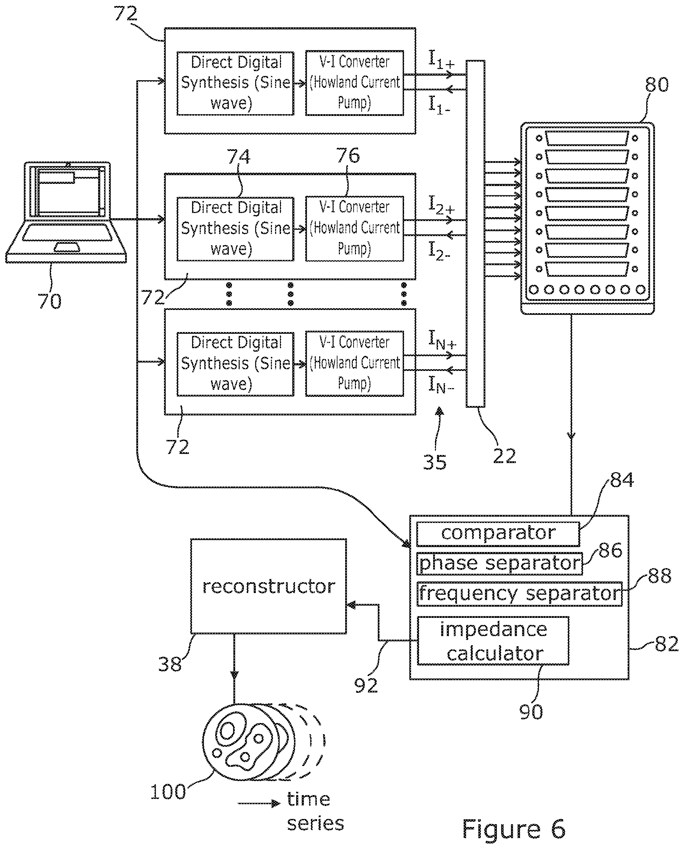

Figure 6 illustrates an arrangement used to implement the techniques discussed above, with functionality generally corresponding to the arrangement of figure 2. A personal computer 70 or other controller is used to control a plurality of signal sources 72, each signal source comprising a direct digital synthesis element 74 for generating a sine wave electrical signal of a chosen frequency and phase. The generated sine wave electrical signal is passed to a Howland current pump 74 acting as a current pump to deliver a current which is the probe electrical signal 35 to and from electrodes of the nerve cuff 22. Not shown in this figure is the switch 32 which is used to change which of the probe electrical signals 35 is connected across which pairs of electrodes 24 at any particular time. The number of signal sources 72 may be selected according to requirements, but six such sources providing three frequencies and two phases in combination may be appropriate. The multiple current sources may be miniaturised and combined using common circuitry to a large extent if required for a miniaturised device.

The electrodes 24 of the cuff 22 are connected to a parallel voltage recorder 80. In figure 6 the parallel voltage recorder 80 records voltages from all the electrodes simultaneously for further analysis by the analyser 82, but if required a more selective collecting and recording of signals may be implemented using the switch 32 mentioned above. The voltages are recorded digitally following analogue to digital conversion in the parallel voltage recorder 80. The recorded voltages may be analysed by the analyser immediately, or recorded for later analysis as required. The voltage recorded may be provided, for example, by an ActiChamp EEG recorder provided by Brain Vision UK, sampling at 100 kHz, with a voltage range of +- 400 mV.

The analyser 80 may typically be implemented in software on a suitable computer system, but hardware implementations, or implementations partly in hardware may be provided, for example using suitable application specific integrated circuits and/or dedicated logic circuitry. The analyser typically implements a number of functional elements or processes, including a comparator arranged to combine recorded voltages, for example in suitable pairs, to form electrical responses corresponding to pairs of electrodes. Such electrical responses may be referred to herein as combined responses 35 in that they contain signals arising from the all of the probe electrical signals being applied at the same time. Such a combined response 35 may then processed by a phase separator function or element 86 and a frequency separator function or element 88 to extract a pair of phase separated electrical responses for each frequency channel.

In practice, although particular comparator, phase separator and frequency separator functions or elements are shown in figure 6, this functionality may typically be implemented in signal processing software in which these functions may be carried out in different ways in combination or separately. For example, the individual frequency components or channels may be separated by software filtering around the injection frequencies of the probe electrical signals with the required bandwidth, for example 3 kHz. Where phase multiplexed probe signals are used at each frequency, a Hilbert transform may be used to provide the phase separation. For example, where two signals are injected with a 90° phase difference (in-phase and quadrature components) the algorithm is:

$$\phi = angle(H_2/H_1)$$

$$V_{ampl} = abs(H_2)$$

$$V_{inphase} = \sqrt{(V_{ampl})^2/(1 + tan(\phi)^2)}$$

$$V_{quadrature} = V_{ampl} \cdot tan(\phi)$$

Here, $H_x$ denotes the Hilbert transform of a given signal. $H_1$ is a base signal for which the phase is known (typically the injection signal) and $H_2$ is the measured signal at the electrode. $\phi$ is the observed phase difference between the signal recorded at the electrode and the original sine signal from the current source.

The phase and frequency separated electrical responses may then used by an impedance calculator function or element 90 to calculate corresponding impedance signals, for example on the basis of data from the computer 70 used to control the signal sources 72, or on the basis of signals from the current sources themselves. In practice, however, the phase and frequency separated electrical responses may be used directly for the tomographic reconstruction, without requiring any intermediate step of overt conversion to impedance.

The determined impedance signals 92 may then be used by a reconstructor element 38 to form one or a series of maps or images of electrical properties of the nerve, for example an electrical impedance tomography image 100 corresponding to a time interval of less than about 5 milliseconds, and preferably less than 1 millisecond, and optionally a series of such images at a rate of at least 200 Hz, and preferably at least 1 kHz.

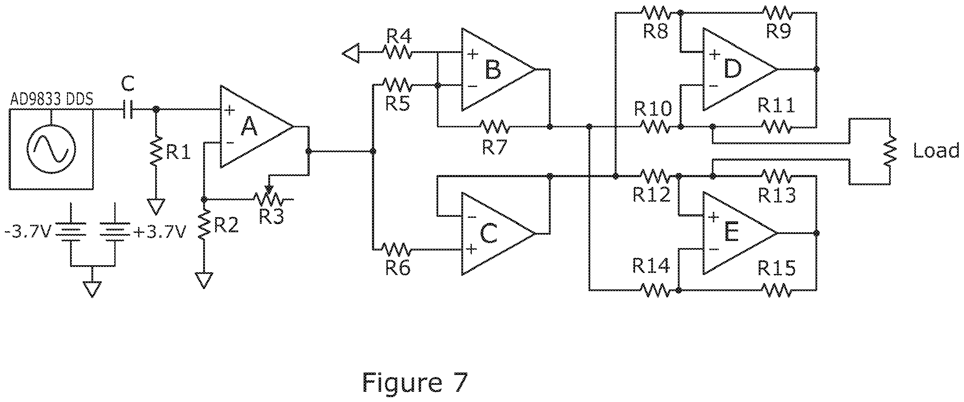

Figure 7 illustrates in more detail an example of how a signal source 72 of figure 6, or of other implementations of the invention, may be provided. An analogue waveform generator such as the Analogue Devices AD9833 device is used to produce a fixed amplitude sine wave with a programmable frequency and phase. Op amp A removes the DC component and amplifies the signal, where the gain can be set by adjusting resistor R3. Single ended to differential conversion is performed by op amps B and C. The final stage including op amps D and E implements a Howland current amplifier which performs voltage to current conversion and is connect to the load, which is provided by a pair of electrodes 24, with the chosen pair being selected and changed for example using the switch 32 already discussed above.

Although particular embodiments of the invention have been described, it will be apparent to the skilled person that various modifications can be made without departing from the scope of the invention.

Although coupling of probe electrical signals into the nerve tissue and collection of electrical responses has been described in terms of electrodes, these do not necessarily need to be in direct contact with the nerve or nerve tissue, and various coupling techniques such as capacitive and inductive coupling may be used, as well as electrical coupling through the skin and/or other tissues.

## Claims

1. Apparatus for determining electrical properties within a peripheral nerve (10) of a human or animal subject, comprising:

   a nerve cuff (22) arranged to space a plurality of electrodes (24) around a perimeter of the peripheral nerve;
   a signal source (34) arranged to simultaneously apply a phase separated pair of first and second probe electrical signals to a set of respective first and second combinations of electrodes, the phase separated pair of first and second probe electrical signals having different signal phases from each other; and
   a detector (36) arranged to receive one or more combined responses to the phase separated pair at one or more of the plurality of electrodes and to detect a pair of phase separated electrical responses corresponding to the different signal phases from the one or more combined responses.

2. The apparatus of claim 1 wherein

   the signal source (34) is arranged to apply each of a plurality of said phase separated pairs of probe electrical signals to a different set of combinations of electrodes (24), such that collecting one or more combined responses comprises collecting one or more combined responses to the phase separated pairs at one or more of the plurality of electrodes, and

the detector (36) is arranged to detect a pair of phase separated electrical responses corresponding to each phase separated pair of probe electrical signals from the one or more combined responses.

3. The apparatus of claim 2 wherein

the signal source (34) is arranged to apply a frequency multiplexed plurality of said phase separated pairs of probe electrical signals simultaneously to each other, each such phase separated pair comprising probe electrical signals of a different frequency band (54), and

the detector (36) is arranged to detect a pair of phase separated electrical responses corresponding to each frequency band (54) from the one or more combined responses.

4. The apparatus of claim 3 wherein the different frequency bands (54) comprise from three to five different frequency bands.

5. The apparatus of claim 4 wherein the centres of all of the frequency bands (54) lie in the range from 2 kHz to 12 kHz.

6. The apparatus of any preceding claim wherein the or each phase separated pair of probe electrical signals comprises first and second probe electrical signals which are substantially in phase quadrature with respect to each other.

7. The apparatus of any preceding claim wherein the nerve cuff (22) comprises:

a flexible substrate for wrapping around at least part of a perimeter of a peripheral nerve (10), and the plurality of electrodes are spaced on the flexible substrate for contacting the peripheral nerve around the perimeter; and

electrical connections (26) to each of the electrodes for applying the probe electrical signals from the signal source, and for collecting the combined responses.

8. The apparatus of any preceding claim wherein the apparatus is arranged to use the or each pair of phase separated electrical responses to determine properties of the peripheral nerve within the perimeter.

9. The apparatus of any preceding claim further comprising a reconstructor (38) arranged to use the or each pair of phase separated electrical responses to determine electrical properties within the peripheral nerve within the perimeter using electrical impedance tomography reconstruction.

10. The apparatus of claim 9 wherein the reconstructor (38) is arranged to determine a map or image of electrical properties of a cross section of the peripheral nerve within said perimeter.

11. The apparatus of any preceding claim wherein the determined electrical properties are representative of impedance.

12. The apparatus of any preceding claim wherein the apparatus is adapted for implantation in the human or animal subject.

**Patentansprüche**

1. Vorrichtung zur Bestimmung elektrischer Eigenschaften innerhalb eines peripheren Nervs (10) eines menschlichen oder tierischen Probanden, umfassend:

eine Nervenmanschette (22), die eingerichtet ist, um eine Mehrzahl von Elektroden (24) um einen Umfang des peripheren Nervs herum beabstandet zu halten;

eine Signalquelle (34), die eingerichtet ist, um gleichzeitig ein phasengetrenntes Paar von ersten und zweiten elektrischen Sondensignalen an einen Satz von jeweiligen ersten und zweiten Kombinationen von Elektroden anzulegen, wobei die ersten und zweiten elektrischen Sondensignale des phasengetrennten Paares voneinander verschiedene Signalphasen aufweisen; und

einen Detektor (36), der eingerichtet ist, um eine oder mehrere kombinierte Antworten auf das phasengetrennte Paar an einer oder mehreren der Mehrzahl von Elektroden aufzufangen und ein Paar phasengetrennter elektrischer Antworten, die den unterschiedlichen Signalphasen entsprechen, aus den ein oder mehreren kombinierten Antworten zu erfassen.

2. Vorrichtung nach Anspruch 1, bei der

die Signalquelle (34) eingerichtet ist, um jedes aus einer Mehrzahl der phasengetrennten Paare von elektrischen Sondensignalen an einen anderen Satz von Kombinationen von Elektroden (24) anzulegen, so dass das Sammeln einer oder mehrerer kombinierter Antworten das Sammeln einer oder mehrerer kombinierter Antworten auf die phasengetrennten Paare an einer oder mehreren aus der Mehrzahl von Elektroden umfasst, und

der Detektor (36) eingerichtet ist, um ein Paar von phasengetrennten elektrischen Antworten zu jedem phasengetrennten Paar von elektrischen Sondensignalen aus den ein oder mehreren kombinierten Antworten zu erfassen.

3. Vorrichtung nach Anspruch 2, bei der

die Signalquelle (34) eingerichtet ist, eine frequenzmultiplexierte Mehrzahl der phasengetrennten Paare von elektrischen Sondensignalen gleichzeitig miteinander anzulegen, wobei jedes solche phasengetrennte Paar elektrische Sondensignale eines anderen Frequenzbandes (54) umfasst, und
der Detektor (36) eingerichtet ist, um zu jedem Frequenzband (54) ein Paar von phasengetrennten elektrischen Antworten aus den ein oder mehreren kombinierten Antworten zu erfassen.

4. Vorrichtung nach Anspruch 3, bei der die verschiedenen Frequenzbänder (54) drei bis fünf verschiedene Frequenzbänder umfassen.

5. Vorrichtung nach Anspruch 4, bei der die Zentren aller Frequenzbänder (54) im Bereich von 2 kHz bis 12 kHz liegen.

6. Vorrichtung nach einem der vorhergehenden Ansprüche, bei der das oder jedes phasengetrennte Paar von elektrischen Sondensignalen ein erstes und ein zweites elektrisches Sondensignal umfasst, die im Wesentlichen in Phasenquadratur zueinander stehen.

7. Vorrichtung nach einem der vorhergehenden Ansprüche, bei der die Nervenmanschette (22) umfasst:

ein flexibles Substrat zum Umwickeln mindestens eines Teils eines Umfangs eines peripheren Nervs (10), wobei die Mehrzahl von Elektroden auf dem flexiblen Substrat beabstandet sind, um den peripheren Nerv um den Umfang herum zu kontaktieren; und
elektrische Verbindungen (26) zu jeder der Elektroden zum Anlegen der elektrischen Sondensignale von der Signalquelle und zum Sammeln der kombinierten Antworten.

8. Vorrichtung nach einem der vorhergehenden Ansprüche, wobei die Vorrichtung eingerichtet ist, um das oder jedes Paar von phasengetrennten elektrischen Antworten zu verwenden, um Eigenschaften des peripheren Nervs innerhalb des Umfangs zu bestimmen.

9. Vorrichtung nach einem der vorhergehenden Ansprüche, die ferner einen Rekonstrukteur (38) umfasst, der eingerichtet ist, um das oder jedes Paar von phasengetrennten elektrischen Antworten zu verwenden, um elektrische Eigenschaften innerhalb des peripheren Nervs innerhalb des Umfangs unter Verwendung von elektrischer Impedanztomographie-Rekonstruktion zu bestimmen.

10. Vorrichtung nach Anspruch 9, bei der der Rekonstrukteur (38) eingerichtet ist, um eine Karte oder ein Bild der elektrischen Eigenschaften eines Querschnitts des peripheren Nervs innerhalb des Umfangs zu bestimmen.

11. Vorrichtung nach einem der vorhergehenden Ansprüche, bei der die ermittelten elektrischen Eigenschaften repräsentativ für die Impedanz sind.

12. Vorrichtung nach einem der vorhergehenden Ansprüche, wobei die Vorrichtung zur Implantation in den menschlichen oder tierischen Probanden eingerichtet ist.

**Revendications**

1. Appareil pour déterminer des propriétés électriques dans un nerf périphérique (10) d'un sujet humain ou animal, comprenant :

un manchon de nerf (22) agencé pour répartir une pluralité d'électrodes (24) autour d'un périmètre du nerf périphérique ;
une source de signaux (34) agencée pour appliquer simultanément une paire séparée en phase de premier et second signaux électriques de sonde à un ensemble de première et seconde combinaisons respectives d'électrodes, la paire séparée en phase de premier et second signaux électriques de sonde ayant des phases de signal différentes l'une de l'autre ; et
un détecteur (36) agencé pour recevoir une ou plusieurs réponses combinées à la paire séparée en phase au niveau d'une ou plusieurs de la pluralité d'électrodes et pour détecter une paire de réponses électriques séparées en phase correspondant aux différentes phases de signal parmi les une ou plusieurs réponses combinées.

2. Appareil selon la revendication 1, dans lequel

la source de signaux (34) est agencée pour appliquer chacune d'une pluralité desdites paires séparées en phase de signaux électriques de sonde à un ensemble différent de combinaisons d'électrodes (24), de sorte que la collecte d'une ou plusieurs réponses combinées comprend la collecte d'une ou plusieurs réponses combinées aux paires séparées en phase au niveau d'une ou plusieurs de la pluralité d'électrodes, et le détecteur (36) est agencé pour détecter une paire de réponses électriques séparées en phase correspondant à chaque paire séparée en phase de signaux électriques de sonde parmi les une ou plusieurs réponses combinées.

3. Appareil selon la revendication 2, dans lequel

la source de signaux (34) est agencée pour appliquer une pluralité multiplexée en fréquence desdites paires séparées en phase de signaux électriques de sonde simultanément les unes aux autres, chacune de ces paires séparées en phase comprenant des signaux électriques de sonde d'une bande de fréquence différente (54), et
le détecteur (36) est agencé pour détecter une paire de réponses électriques séparées en phase correspondant à chaque bande de fréquence (54) parmi les une ou plusieurs réponses combinées.

4. Appareil selon la revendication 3, dans lequel les différentes bandes de fréquence (54) comprennent de trois à cinq bandes de fréquence différentes.

5. Appareil selon la revendication 4, dans lequel les centres de toutes les bandes de fréquence (54) se situent dans la plage de 2 kHz à 12 kHz.

6. Appareil selon l'une quelconque des revendications précédentes, dans lequel la ou chaque paire séparée en phase de signaux électriques de sonde comprend des premier et second signaux électriques de sonde qui sont sensiblement en quadrature de phase l'un par rapport à l'autre.

7. Appareil selon l'une quelconque des revendications précédentes, dans lequel le manchon de nerf (22) comprend :

un substrat flexible pour s'enrouler autour d'au moins une partie d'un périmètre d'un nerf périphérique (10), et la pluralité d'électrodes sont réparties sur le substrat flexible pour entrer en contact avec le nerf périphérique autour du périmètre ; et
des connexions électriques (26) à chacune des électrodes pour appliquer les signaux électriques de sonde provenant de la source de signaux, et pour collecter les réponses combinées.

8. Appareil selon l'une quelconque des revendications précédentes, dans lequel l'appareil est agencé pour utiliser la ou chaque paire de réponses électriques séparées en phase pour déterminer des propriétés du nerf périphérique à l'intérieur du périmètre.

9. Appareil selon l'une quelconque des revendications précédentes comprenant en outre un reconstructeur (38) agencé pour utiliser la ou chaque paire de réponses électriques séparées en phase pour déterminer des propriétés électriques à l'intérieur du nerf périphérique à l'intérieur du périmètre en utilisant une reconstruction tomographique d'impédance électrique.

10. Appareil selon la revendication 9, dans lequel le reconstructeur (38) est agencé pour déterminer une carte ou une image de propriétés électriques d'une coupe transversale du nerf périphérique à l'intérieur dudit périmètre.

11. Appareil selon l'une quelconque des revendications précédentes, dans lequel les propriétés électriques déterminées sont représentatives d'une impédance.

12. Appareil selon l'une quelconque des revendications précédentes, dans lequel l'appareil est adapté pour une implantation chez le sujet humain ou animal.

Figure 1

Figure 2

Figure 3

Figure 4

Figure 5

72

Direct Digital Synthesis (Sine wave) | V-I Converter (Howland Current Pump)

$I_{1+}$

$I_{1-}$

80

74    76

Direct Digital Synthesis (Sine wave) | V-I Converter (Howland Current Pump)

$I_{2+}$

$I_{2-}$

70

72

Direct Digital Synthesis (Sine wave) | V-I Converter (Howland Current Pump)

$I_{N+}$

$I_{N-}$

72

35    22

84

comparator

phase separator    86

frequency separator    88

reconstructor

impedance calculator    82

38    92    90

100

time series

Figure 6

Load

R9  R11  R8  R10  D

R13  R15  R12  R14  E

R7  B  C  R4  R5  R6

A  R3  R2  R1  C

AD9833 DDS

+3.7V  -3.7V

Figure 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016170327 A
- EP 2543311 A

- WO 2017030900 A

**Non-patent literature cited in the description**

- **D.S.HOLDER.** *Medical & Biological Engineering & Computing,* 30 March 1992, 140-146